# EUROPÄISCHE PATENTSCHRIFT

(11) **EP 2 816 951 B1**
(45) Veröffentlichungstag und Bekanntmachung des Hinweises auf die Patenterteilung: **03.02.2016**
(21) Anmeldenummer: 13810883.2
(22) Anmeldetag: 15.11.2013
(51) Int. Cl.: A61B 5/055, A61G 13/12

(54) **VORRICHTUNG ZUR INSBESONDERE TRANSPERINEALEN UNTERSUCHUNG VON PATIENTEN**
APPARATUS FOR THE IN PARTICULAR TRANSPERINEAL EXAMINATION OF PATIENTS
DISPOSITIF SERVANT, EN PARTICULIER, À L'EXAMEN TRANSPÉRINÉAL DE PATIENTS

(30) Priorität: 23.11.2012 DE 102012022834
(43) Veröffentlichungstag der Anmeldung: 31.12.2014
(73) Patentinhaber: Noras, Hubert, 97082 Würzburg (DE)
(72) Erfinder: Noras, Hubert, 97082 Würzburg (DE)
(74) Vertreter: Pöhner, Wilfried Anton
(86) Internationale Anmeldenummer: PCT/DE2013/100385
(87) Internationale Veröffentlichungsnummer: WO 2014/079415

(56) Entgegenhaltungen:
- WO-A1-2009/000321
- US-A- 5 871 448
- US-A- 5 957 935
- US-A1- 2004 133 979
- US-A1- 2008 216 239

## Beschreibung

Die Erfindung bezieht sich auf die Schaffung einer Vorrichtung zur insbesondere transperinealen Untersuchung von Patienten gemäß dem Oberbegriff des Anspruches 1.

Das im Zusammenhang mit vorliegender Anmeldung vornehmlich behandelte Themengebiet ist das der optimalen Erfassung von Erkrankungen der Prostata, insbesondere des Prostatakarzinoms durch bildgebende Verfahren, die Entnahme von Gewebeproben durch Biopsieverfahren und die Vornahme und das Durchführen operativer Behandlungsmaßnahmen unterschiedlichster Art. Wesentliche Voraussetzung zur Optimierung der einschlägig bekannten Verfahren ist, optimale Voraussetzungen dafür zu schaffen, um die vorbeschriebenen Schritte (Durchführung bildgebender Verfahren, Biopsie, operative Eingriffe zu therapeutischen Zwecken) durchführen zu können.

Im Zusammenhang mit einer jüngst erschienenen Veröffentlichung in Eur Radiol (2012) 22:1829 - 1835 (European Society of Radiology 2012) ist eine Vorrichtung beschrieben, bei welcher der Patient auf einer Liege in die NNMR-Röhre eingefahren wird, die zwei Beinstützen aufweist, der Patient mithin auf der Vorrichtung liegend sich in einer Position befindet, die vergleichbar ist den bekannten Anordnungen auf einem gynäkologischen Stuhl, mit dem wesentlichen Unterschied jedoch, dass der Stuhl um 90 ° nach hinten verkippt ist, d.h. die Stuhllehne sich in der Horizontalen befindet und durch einen Schlitten in die Röhre eingefahren wird. Die Beine werden im Bereich artige Behandlungsstühle sind hinausgehend über den Bereich der Gynäkologie auch auf dem Fachgebiet der Urologie allgemein geläufig.

Die Schaffung optimaler Arbeitsbedingungen ist Voraussetzung für die Realisierung bestmöglicher Arbeitsergebnisse. Hierzu zählt, dass der Dammbereich, der sich als jener Bereich des Beckens, der sich zwischen den Oberschenkeln befindet definiert, möglichst weiträumig zugänglich ist, um das Einbringen von Spulen und/oder die Entnahme der Gewebeproben durch Einführen von Biopsienadeln und/oder der chirurgischen Werkzeuge möglichst günstig durchführen zu lassen. Die Lösung dieses Problemes gestaltet sich bei adipösen Patienten und insbesondere solchen mit großem Durchmesser der Oberschenkel als problematisch. Im Bereich der Gynäkologie als auch dem der Urologie wird dieses Problem dadurch gelöst, dass die Oberschenkel durch Spreizung der Beinhalterungen soweit geöffnet werden, d.h. der Öffnungswinkel hinreichend groß gewählt wird, um die angestrebte optimale Zugänglichkeit zu erhalten. Durch die Möglichkeit, den Öffnungswinkel der Beine beliebig zu gestalten, lassen sich auch bei extrem starken Oberschenkeln hinreichend zufriedenstellende Arbeitsbedingungen realisieren, um den Dammbereich zur Vornahme der vorgeschilderten Maßnahmen in ausreichendem Maße zugänglich zu gestalten.

Derartige Vorrichtungen und die Wahl auch größerer Öffnungswinkel lassen sich in vielen Fällen nicht umsetzen, da die entsprechenden vorzunehmenden Maßnahmen in einem NMR- Tomographiegerät üblicher Bauart vorgenommen werden müssen und durch die röhrenförmige Ausbildung, in der der Patient während der diagnostischen und therapeutischen Maßnahmen einzubringen ist, die Möglichkeit der Öffnung der Oberschenkel durch die Geometrie der Röhre des NMR-Gerätes limitiert ist.

Die üblichen Untersuchungsmethoden bei Verdacht auf Prostatakarzinom ist wie folgt: Grundlage und Anlass für eine genauere Untersuchung ist das Vorliegen eines erhöhten PSA-Wertes, der als Verdacht auf das Vorliegen eines Prostatakarzinoms zu werten ist. Herkömmlicherweise besteht der nächste Schritt dann darin, im Wege der Biopsie Gewebeproben zu entnehmen und sie histologisch zu untersuchen, um den Verdacht zu bestätigen oder zu revidieren. Problematisch ist, dass auf der Grundlage von Ultraschalldiagnostik eine Bestimmung des Ortes und der regionalen Ausdehnung des verdächtigen Gewebes nicht sicher von gesundem Gewebe abgegrenzt werden kann, sodass die Entnahme mehrerer Gewebeproben erforderlich wird. Ein wesentlicher Fortschritt bringt hier die Anwendung von bildgebenden Verfahren auf der Grundlage der NMR-Technik, die eine sichere Abgrenzung und Identifizierung des zu biopsierenden verdächtigen Gewebes von dem gesunden möglich macht. Aus diesem Grunde gehört es zur aktuellsten und modernsten Vorgehensweise, unter einer NMR-gesteuerten Prostatabiopsie die verdächtigen Gewebe zu entnehmen. Es bedarf keiner näheren und eingehenden Begründung, weshalb die Vornahme mehrerer Biopsien von erheblichem Nachteil anzusehen sind.

Dem Erfindungsgegenstand kommt die US 2008/216239 A1 am nächsten, die einen Tisch zur Durchführung medizinischer Untersuchungen, bei dem der Patient auf dem Rücken liegend seine Unterschenkel auf Beinhalterungen des Tisches auflegt. Auf Beinhöhe in Richtung der Mittelebene des Patienten bewegbar findet sich ein Schlitten, der einen Rahmen trägt, in dem entweder ein Gitter und/oder einer Zielvorrichtung und/oder einer NNMR-Spule einbringbar ist. Bauliche Maßnahmen zur Spreizung der Oberschenkel sind nicht vorgesehen. Gleiches gilt auch für die WO 2009/000321 A1, die in ihrem Offenbarungsgehalt insoweit übereinstimmt.

Ein weiteres Gerät zur Durchführung von medizinischen Untersuchungen ist aus der US 5 871 448 A bekannt, wo jedoch ein Bezug zum Patienten sowie dessen Fixierung vollständig fehlen.

Die US 2004/133979 A1 schließlich zeigt einen Patiententisch für chirurgische Untersuchungen, durch welche der Patient in unterschiedlichsten Positionierungen fixierbar ist. In einem Spezialfall wird ein Sattel gezeigt, der an der Innenfläche des Oberschenkels anliegt und an einer Sitzvorrichtung angebracht ist. Eine Bewegung der Schenkel des Sattels in der durch die beiden Oberschenkel des Patienten definierten Ebene nach außen findet nicht statt.

Hiervon ausgehend hat sich die Erfindung die Schaffung einer Vorrichtung zur Aufgabe gemacht, mit deren Hilfe bei Patienten, insbesondere solchen mit korpulenten Oberschenkeln, die Durchführung von Untersuchungen und Behandlungen vor allem durch Verbesserung der Zugänglichkeit des Dammbereiches wesentlich erleichtert werden kann.

Gelöst wird diese Aufgabe durch die im Anspruch 1 angegebenen Merkmale.

Die erfindungsgemäß vorgeschlagene Vorrichtung besteht in ihrem grundsätzlichen Aufbau aus drei Elementen. Zum einen ist eine Beinhalterung vorhanden, die zur Aufnahme des Unterschenkels des Patienten nach Art einer Halbschale gestaltet ist, sowie einem Fuß, auf dem die Halbschale ruht. Symmetrisch zu dieser beschriebenen Beinhalterung existiert als 2. Element eine weitere Beinhalterung von dem wesentlichen gleichen Aufbau, die symmetrisch zur ersten Beinhalterung angeordnet ist. Die Symmetrieachse beschreibt die Längsachse des Patienten. Als Drittes schließlich ist ein in Richtung der Symmetrieachse im Wesentlichen in horizontaler Richtung verfahrbarer Schlitten vorhanden, an dessen oberen, d.h. patientennahen Ende, ein Rahmen befestigt ist. Dabei ist der Rahmen so positioniert, dass er in jenem Beckenbereich, der sich zwischen den Innenflächen der Oberschenkel befindet, anzuliegen kommt. Die Anwendung der vorgeschlagenen Vorrichtung ist damit vornehmlich für transperinealen Anwendung vorgesehen, was in speziellen Fällen jedoch die Verwendung transgluteale oder transrektale Eingriffe nicht grundsätzlich ausschließt. Damit eignet sich die erfindungsgemäß vorgeschlagene Vorrichtung auch für gynäkologische Zwecke und für Untersuchungen des Enddarmes. Als wesentlicher Vorteil des transperinealen Zugriffes ist zu sehen, dass die Gabe von Antibiotika, wie sie bei Biopsien durch den Darm (transrektaler Zugang) zwingend erforderlich sind, überflüssig ist.

In einer Weiterbildung der erfindungsgemäßen Vorrichtung, um bei allen Patienten und insbesondere bei solchen mit korpulenten Oberschenkeln die Zugänglichkeit des Dammbereiches zur Durchführung der diagnostischen und/oder therapeutischen Maßnahmen zu verbessern, wird deshalb vorgeschlagen, dass zweiseitige Anpressbacken in die Vorrichtung integriert werden, die symmetrisch zu der durch die Patienten längsachse definierte Symmetrieachse angeordnet sind und sich in Richtung auf den Patienten bewegen und sich abstandsmäßig verringern lassen. Die beiden Anpressbacken beschreiben demnach im Hinblick auf ihre räumliche Anordnung, die beiden Schenkel eines gleichschenkligen Trapezes. Dabei wird, entsprechend der üblichen Terminologie der Geometrie, ein Trapez als ein ebenes Viereck beschrieben mit zwei parallelen, ungleich langen Seiten, den Grundlinien, und zwei nicht parallelen Seiten, die als Schenkel bezeichnet sind. Bei identischer Länge beider Schenkel spricht man von einem gleichschenkligen Trapez.

Die Anwendung geschieht in der Weise, dass mit Erreichen der Endposition die Backen im Wesentlichen in der durch den Patientendefinierten Ebene senkrecht zur Längsachse des Patienten nach außen zu bewegt und an die Oberschenkel angepresst werden. In Abhängigkeit von der Anatomie und dem beaufschlagten Anpressdruck, wird die Innenseite, genauer die elastischen Volumenbereiche des Oberschenkels, die sich im Wesentlichen durch die Fett- und Muskelbestandteile bestimmen, im Hinblick auf die durch den Patienten gebildete Ebene im Wesentlichen nach oben und unten, d.h. senkrecht zu dieser Ebene, deformieren, dass eine wesentliche Verbesserung des Zuganges zum Damm resultiert. Unter Beibehaltung der Position der Längsachse der Oberschenkel bewirken die Anpressbacken eine deutliche Vergrößerung des für den Zugang zum Dammbereich zur Verfügung stehenden Raumvolumens. Hierdurch lässt sich eine spürbare Verbesserung des Handlings von Diagnose- und/oder Therapieinstrumenten erreichen, demzufolge eine Verbesserung der Arbeitsergebnisse zu erwarten steht.

Die Art und Weise der Verstellung der Anpressbacken, d.h. deren Anfahren und Anpressen gegen die Innenseite des Oberschenkels kann auf unterschiedliche Weise erfolgen. Befindet sich der Patient in seiner Endposition, in der die Unterschenkel auf den Beinhalterungen aufliegen, werden die Anpressbacken gegen den Unterschenkel gefahren. Vom Grundprinzip her ist es unerheblich, ob ein sukzessives Heranfahren der einzelnen Anpressbacken an den Oberschenkel erfolgt, oder ob dies in einer synchronen und auch symmetrisch zur Längsachse des Patienten symmetrischen Bewegung erfolgt. Hieraus folgt selbstredend, dass während der Phase, in der der Patient in die Endposition verbracht wird und auch, so lange der Rahmen noch nicht an den Damm verfahren und dort angepresst wird, die beiden Backen relativ nahe zusammen stehen.

Der Rahmen ist so gestaltet, dass er in der Lage ist, verschiedene Elemente aufzunehmen und deren Austauschen zu gestatten. Hierzu zählen ausdrücklich Gitter, bei deren Verwendung zum einen eine Fixierung des Probanten erfolgt und zum anderen durch die gitterförmigen Öffnungen die Möglichkeit besteht, durch Biopsienadeln durch das Gitter hindurch geführt und positionsgenau einzustechen. Die Vorgehensweise ist meist wie folgt: Zunächst werden durch NMR-Techniken die Lage des verdächtigen Gewebes ermittelt und exakt ausgemessen und die Koordinaten bestimmt. Die ermittelten Koordinaten werden dazu benutzt, jene Gitteröffnung zu bestimmen, durch welche das medizinische Werkzeug, so z.B. die Biopsienadel, durchgestochen werden muss. Die Koordinaten können weiterhin dazu dienen, die Einstechtiefe des Werkzeuges zu ermitteln. Diese Vorgehensweise erlaubt es, den Eingriff auf die verdächtigen Gewebebereiche zu beschränken und gezielt Gewebe zu entnehmen (Biopsie) und/oder operative Eingriffe durchzuführen.

Unabhängig davon kann bei der Anwendung von NMR-Verfahren zu rein bildgebenden Zwecken in den Rahmen eine Spule eingesteckt werden, die zu einer wesentlichen Verbesserung der Bildqualität beitragen wird.

Der den Rahmen tragende Schlitten erlaubt es, den Patienten ungehindert in die Behandlungsposition zu verbringen, d.h. den Patienten auf den Rücken zu legen, auszurichten und die Unterschenkel in die dafür bestimmte Beinhalterung aufzulegen. Nachdem sich während dieser Phase der Schlitten in zurückgezogener, d.h. patientenfernen Position befindet, ist dies ohne Behinderungen möglich. Befindet sich der Patient dann in der Endposition, wird der Schlitten herangefahren und zwar soweit, bis der Rahmen mit den darin befindlichen Gittern oder Spulen aufgrund translatorischer Verschiebung im Dammbereich zur Anlage kommt. Dort wird dann der Schlitten für die Dauer der Untersuchung fixiert.

Die bauliche Gestaltung des Schlittens in seinem grundsätzlichem Aufbau besteht aus Schienen, die in Richtung der Mittelachse des Patienten ausgerichtet sind und auf denen sich der Schlitten mit dem daran befestigen und etwa auf die Mitte ausgerichteten Rahmen befindet. Zur temporären Festlegung des Schlittens sind Fixiermittel vorgesehen.

Die vorgeschlagene Vorrichtung erweist sich von Vorteil bei der Entnahme histologisch zu untersuchenden Gewebe (Biopsien), beim Entfernen von Karzinomen nach unterschiedlichen operativen Methoden und zur Verbesserung der Bildqualität bei NMR -Aufnahmeverfahren. Ohne Beschränkung der Allgemeinheit können bei den operativen Methoden Kryo-Verfahren eingesetzt werden, die sich dadurch charakterisieren, dass im Bereich des Karzinoms Einstiche in hinreichender Zahl eingebracht werden, sodass es nach dem Einbringen von Kältemittel zu einer Zerstörung des karzinogenen Gewebes kommt.

Die bauliche Gestaltung des Schlittens kann dadurch erfolgen, dass in zwei einander gegenüberliegenden Randbereichen jeweils wenigstens zwei Führungsflügel angeordnet sind, die in U-förmig gestalteten Schienen eingreifen. Durch Klemmschrauben kann das vorübergehende Festlegen des Schlittens erfolgen.

Als vorteilhaft wurde erkannt, dass die gesamte, aus den beiden Beinhalterungen und dem Schlitten bestehende Vorrichtung einstückig ist. Bei der Ausrichtung und Justierung, aber auch beim Verstellen von Beinhalterungen und Schlitten, lassen sich die symmetrische Zuordnung und synchrone Verstellung am einfachsten umsetzen.

Grundsätzlich gilt schließlich, dass die Beinhalterungen, zur Anpassung an den unterschiedlichen anatomischen Gegebenheiten, Verstellmöglichkeiten vorsehen.

Die Verstellbarkeit des Rahmens und demzufolge der darin aufzunehmenden Gitter und Spule erlaubt nicht nur die Anpassung an die unterschiedlichen anatomischen Gegebenheiten, sondern erlaubt ohne Weiteres abgehend von der im Vordergrund stehend transperinealen Zugänglichkeit der Prostata auch Untersuchungen des gynäkologischen Bereiches oder des Darmes vorzunehmen. Ausdrücklich als bevorzugt wird angesehen, wenn das Heranbringen und Anlegen des Rahmens an den Dammbereich durch Verfahren eines Schlittens auch gleichzeitig dazu benutzt wird, um die Anpressbacken in Bezug auf die Mittelachse nach außen zu fahren. Hierzu ist vorgesehen, dass der Schlitten eine mechanische Verbindung mit den die Verschwenkung der Anpressbacken bewirkenden Betätigungshebeln aufweist. In einem solchen Fall sind alle Bewegungen, nämlich das Anpressen des Rahmens an den Dammbereich sowie das Verfahren beider Anpressbacken nach außen synchron, sodass die Zeiten der Vorbereitungsarbeiten minimiert werden können.

Um das symmetrische Auseinanderfahren der Anpressbacken baulich umzusetzen, ist bevorzugt eine Scherenmechanik vorzusehen, die die Durchführung einer symmetrischen Bewegung sicherstellt.

Als Antrieb für die Scherenmechanik ist jede Art von Antrieb denkbar und insbesondere auch, dass die Bewegung des Schlittens zur Betätigung der Scherenmechanik genutzt wird.

Weitere Einzelheiten, Merkmale und Vorteile der Erfindung lassen sich demnach folgenden Beschreibungsteil entnehmen, in dem anhand der Zeichnung ein Ausführungsbeispiel der Erfindung näher erläutert wird. Es zeigen:

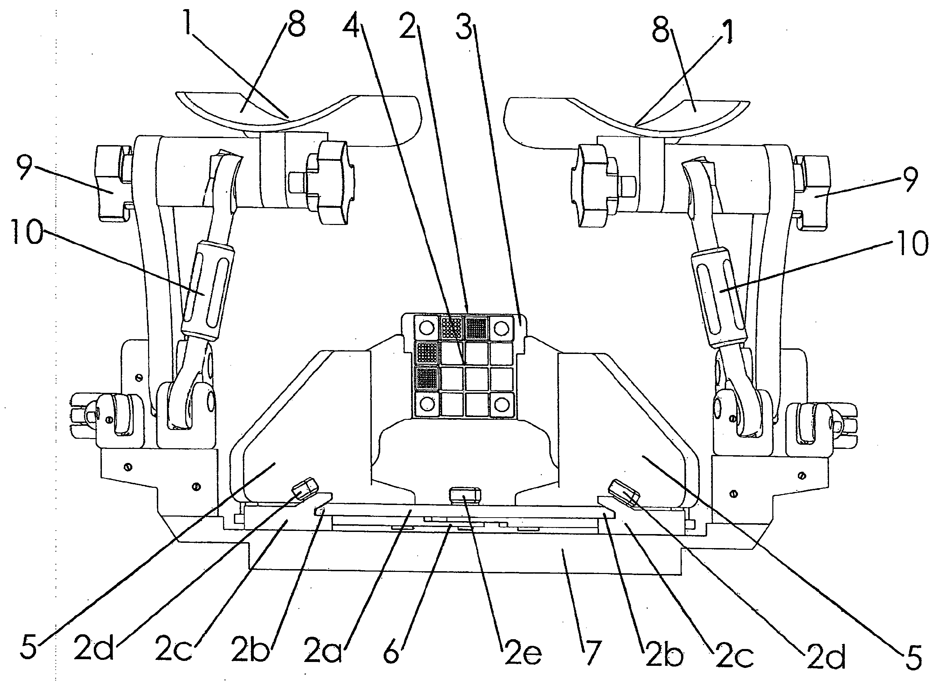

- Figur 1: die erfindungsgemäße Vorrichtung in Längsrichtung des Schlittens.

Die in schematischer gehaltene Draufsicht auf die erfindungsgemäße Vorrichtung erfolgt in Bewegungsrichtung des Schlittens und ohne Patient.

Die Vorrichtung wird im Wesentlichen charakterisiert durch zwei Beinhalterungen gleichen Aufbaus, die aufeinander zugerichtet sind und symmetrisch zu der senkrecht zur Zeichenebene verlaufenden Mittelebene ausgerichtet sind. Dazwischen befindet sich und zwar ebenfalls symmetrisch zu dieser Mittelebene ein Schlitten 2 mit dem daran befestigen Rahmen 3, in dem in dem gezeigten Ausführungsbeispiel ein Gitter 4 eingesteckt ist.

Der Schlitten 2 besteht im gezeigten Ausführungsbeispiel aus einer horizontal verlaufenden Platte 2a, die an ihren beiden randseitigen und einander gegenüberliegenden Enden mit Führungsflügel 2b versehen sind, die ihrerseits jeweils in senkrecht zur Zeichenebene verlaufenden U-förmigen Schienen 2c eingreifen. Zur Fixierung der Schlittenbewegung, wie sie am Anschlagspunkt des Rahmens am Perinealbereich erforderlich sind, befinden sich im Bereich der Schiene an jeder Seite jeweils zwei Fixierschrauben 2d, welche die Schiene 2c durchgreifen und in der Lage sind die Führungsflügel festzuklemmen. Unterhalb der Platte 2a befindet sich eine Scherenmechanik 6 angedeutet, deren einer Drehpunkt der Befestigungspunkt 2e der Scherenmechanik 6 an der Platte 2a ist. Diese Scherenmechanik 6 bewirkt, dass sich bei einem Verschieben des Schlittens 2 in der gezeigten Anordnung bei einer Bewegung auf den Patienten zu die Anpressbacken 5 symmetrisch nach außen zu bewegen und bei einem Zurückfahren sich die Anpressbacken 5 wieder aufeinander zu bewegen.

Die gesamte soeben beschriebene Anordnung findet sich auf einer gemeinsamen Bodenplatte 7 fixiert.

Die konkrete Ausgestaltung der Beinhalterung 1 ist aus dem Stande der Technik bekannt. An seiner Oberseite findet sich jeweils eine wannenförmige Auflagefläche 8, die so zu dimensionieren ist, dass sie in etwa an die Oberflächenform des Unterschenkels angepasst sind. Diese Auflagefläche 8 ist um die horizontale, in der Zeichenebene verlaufende Achse verschwenkbar und über Arretiermutter 9 festlegbar.

Die gesamte Anordnung befindet sich an einem Hebel, der im Bereich der Bodenplatte 7 verschwenkbar befestigt ist und somit eine Anpassung an unterschiedliche Beinlängen erlaubt. Die Einstellung des Winkels zur Längsachse des Patienten erfolgt mittels einer Verschwenkung um die vertikale Achse, die im gezeigten Ausführungsbeispiel dadurch erfolgt, dass ein mit einem Ende ortsfest angebrachter und in vorliegender Zeichnung verdeckten Zylinder an einem exzentrisch zur Vertikalachse befindlichen Punkt angreift. Die Betätigung des Zylinders führt zu einer Verschwenkung des Schwenkhebels um die vertikale Achse.

Zur Abstützung jedes Beinhalters 1 ist ein mit seinen beiden Eckpunkten verschwenkbar befestigter Zylinder 10 angebracht. Die schwenkbare Anordnung erlaubt unter Beibehaltung der Abstützung eine Justierung des Beinhalters, die selbstredend in der Art und Weise zu erfolgen hat, dass sie den körperlichen Proportionen des Patienten individuell angepasst werden.

Im Ergebnis erhält man eine Vorrichtung, die sich in besonderer Weise für den Einsatz von Untersuchungen der Prostata und insbesondere des Prostatakarzinoms eignet, wobei sich die Behandlung und sowohl zur Entnahme histologisch zu untersuchendem Gewebe im Rahmen einer Biopsie, aber auch eines operativen Eingriffes, zur Beseitigung des karzinogenen Gewebes eignet. Im Falle der Einbringung von Aufnahmespulen in den Rahmen lässt sich eine verbesserte Bildqualität erreichen. Es bedarf keiner eingehender Erläuterungen, dass sich diese Vorrichtung auch dazu eignet, in der Gynäkologie oder zur Durchführung rektaler Operationen, wobei sich die Verstell- bzw. Justierbarkeit des Rahmens als besonderer Vorteil herausstellt.

### Bezugszeichenliste

- 1: Beinhalterung
- 2: Schlitten
2a Platte
2b Führungsflügel
2c U-förmige Schiene
2d Fixierschraube
2e Befestigungspunkt der Scherenmechanik
- 3: Rahmen
- 4: Gitter
- 5: Anpressbacke
- 6: Scherenmechanik
- 7: Bodenplatte
- 8: wannenförmige Auflagefläche
- 9: Arrtiermutter
- 10: Zylinder

## Patentansprüche

1. Vorrichtung zur insbesondere transperinealen Untersuchung von Patienten, bestehend aus zwei symmetrisch zur Längsachse des Patienten angeordneten Beinhalterungen (1), wobei zwischen den Beinhalterungen (1) ein in Richtung der Längsachse verschieb- und arretierbarer und in einer parallel zur Patientenmittelebene verlaufender Schlitten (2) angeordnet ist, der an seinem patientenseitigen Ende einen senkrecht zur Verschieberichtung orientierten Rahmen (3) aufweist, der zur Aufnahme eines Gitters (4) und/oder einer Zielvorrichtung oder einer NNMR-Spule geeignet ist, **dadurch gekennzeichnet, dass** zwei Anpressbacken (5) vorhanden sind, die symmetrisch zur Längsachse des Patienten und im spitzen Winkel auf den Patienten zu ausgerichtet sind und in der durch die beiden Oberschenkel des Patienten definierten Ebene nach außen zu bewegbar sind.

2. Vorrichtung nach Anspruch 1, **dadurch gekennzeichnet, dass** das Gitter (4) und/oder die Zielvorrichtung und/oder die NNMR-Spule in den Rahmen einsteck- und wieder herausnehmbar ist.

3. Vorrichtung nach Anspruch 1 oder 2, **dadurch gekennzeichnet, dass** der Rahmen (3) und/oder die Beinhalter (1) in ihrer räumlichen Ausrichtung justierbar sind.

4. Vorrichtung nach einem der Ansprüche 1 bis 3, **dadurch gekennzeichnet, dass** der Schlitten (2) entlang von parallel zur Patientenmittelebene verlaufenden Schienen (2c) bewegbar und in der Anschlagsposition des Rahmens (2) am Damm fixierbar ist.

5. Vorrichtung nach einem der Ansprüche 1 bis 4, **dadurch gekennzeichnet, dass** der Schlitten (2) seitliche Führungsflügel (2b) aufweist, die horizontal in U-förmigen Schienen (2c) eingreifen.

6. Vorrichtung nach einem der Ansprüche 1 bis 5, **dadurch gekennzeichnet, dass** die beiden Beinhalterungen (1) und der Schlitten (2) miteinander verbunden, insbesondere einstückig sind.

7. Vorrichtung nach Anspruch 1, **dadurch gekennzeichnet, dass** die beiden Anpressbacken (5) durch einen gemeinsamen Antrieb synchron nach außen bewegbar sind.

8. Vorrichtung nach Anspruch 1 oder 7, **dadurch gekennzeichnet, dass** die Anpressbacken (5) mit einer Scherenmechanik (6) in Verbindung stehen.

9. Vorrichtung nach einem der Ansprüche 1 bis-8, **dadurch gekennzeichnet, dass** der Antrieb der Anpressbacken (5) durch Kopplung an den Schlitten (2) gebildet ist.

## Claims

1. Device for the, in particular, transperineal examination of patients, consisting of two leg holders (1), which are arranged symmetrically to the longitudinal axis of the patient, wherein, between the leg holders (1), there is disposed a carriage (2), which extends in the direction of the longitudinal axis such that it is displaceable parallel to the patient's centre plane and lockable, and which has at its end close to the patient a frame (3) which is oriented perpendicular to the displacement direction and is suitable for receiving a grid (4) and/or a target device or an NNMR coils, **characterised in that** two pressing jaws (5) are present, which are oriented symmetrically to the longitudinal axis of the patient and at an acute angle to the patient and can be moved outwardly in the plane defined by the two thighs of the patient..

2. Device according to claim 1, **characterized in that** the grid (4) and/or the target device and/or the NNMR coil can be plugged into the frame and removed from it again.

3. Device according to claim 1 or 2, **characterized in that** the frame (3) and/or the leg holder (1) are adjustable in their spatial orientation.

4. Device according to one of claims 1 to 3, **characterized in that** the carriage (2) is movable along rails (2c) which extend parallel to the centre plane of the patient and can be fixed in the position at which the frame (2) strikes against the perineum.

5. Device according to one of claims 1 to 4, **characterised in that** the carriage (2) has lateral guide wings (2b), which engage horizontally in U-shaped rails (2c).

6. Device according to one of claims 1 to 5, **characterised in that** the two leg holders (1) and the carriage (2) are integrated with one another, in particular as one piece.

7. Device according to claim 1, **characterised in that** the two pressing jaws (5) are movable synchronously outwardly by means of a common drive.

8. Device according to claim 1 or 7, **characterized in that** the pressing jaws (5) are in connection with a scissor mechanism (6).

9. Device according to one of claims 1 to 8, **characterised in that** the drive of the pressing jaws (5) is formed by coupling to the carriage (2).

## Revendications

1. Dispositif destiné notamment à l'examen transpérinéal de patients, consistant en deux supports de jambe (1) disposés de façon symétrique par rapport à l'axe longitudinal du patient, sachant qu'un berceau (2) pouvant être déplacé et arrêté dans le sens de l'axe longitudinal et courant dans un plan parallèle au plan médian du patient est disposé entre les supports de jambe (1), lequel présente à son extrémité située du côté du patient un cadre (3) orienté verticalement par rapport au sens du déplacement, qui est capable de recevoir une grille (4) et/ou un dispositif cible ou une bobine NNMR, **caractérisée par le fait qu'**il existe deux mâchoires de pressage (5) orientées symétriquement par rapport à l'axe longitudinal du patient et formant un angle aigu par rapport au patient, lesquelles peuvent être déplacées vers l'extérieur dans le plan défini par les parties supérieures des cuisses du patient.

2. Dispositif selon la revendication 1, **caractérisé par le fait que** la grille (4) et/ou le dispositif cible et/ou la bobine NNMR peut être enfiché ou retiré dans le cadre.

3. Dispositif selon la revendication 1 ou 2, **caractérisé par le fait que** le cadre (3) et/ou les supports de jambe (1) peuvent être ajustés dans leur orientation spatiale.

4. Dispositif selon une des revendications 1 à 3, **caractérisé par ²²le fait** que le berceau (2) peut être déplacé le long de rails (2c) et peut être fixé dans la position de butée du cadre (2) sur la digue.

5. Dispositif selon une des revendications 1 à 4, **caractérisé par le fait que** le berceau (2) présente des ailes de guidage latérales (2b) qui s'engrènent horizontalement dans les rails (2c) en forme de U.

6. Dispositif selon une des revendications 1 à 5, **caractérisé par le fait que** les deux supports de jambe (1) et le berceau (2) sont reliés l'un à l'autre, et sont notamment en une pièce.

7. Dispositif selon la revendication 1, **caractérisé par le fait que** les deux mâchoires de pressage (5) peuvent être déplacées de façon synchrone vers l'extérieur par un entraînement commun.

8. Dispositif selon une des revendications 1 à 7, **caractérisé par le fait que** les deux mâchoires de pressage (5) sont reliées à un dispositif mécanique de ciseaux (6).

9. Dispositif selon une des revendications 1 à 8, **caractérisé par le fait que** l'entraînement des mâchoires de pressage (5) est constitué d'un couplage sur les berceaux (2).
